(19) Europäisches Patentamt
European Patent Office
Office européen des brevets

(11) **EP 4 633 473 B1**

(12) **EUROPEAN PATENT SPECIFICATION**

(45) Date of publication and mention
of the grant of the patent:
**21.01.2026 Bulletin 2026/04**

(21) Application number: **23817057.5**

(22) Date of filing: **29.11.2023**

(51) International Patent Classification (IPC):
*A61B 6/06* *(2006.01)* *A61B 6/00* *(2024.01)*
*G21K 1/04* *(2006.01)*

(52) Cooperative Patent Classification (CPC):
**A61B 6/06; A61B 6/488; A61B 6/542; A61B 6/545;**
A61B 6/5217

(86) International application number:
**PCT/EP2023/083443**

(87) International publication number:
**WO 2024/126037 (20.06.2024 Gazette 2024/25)**

(54) **CONTROLLED COLLIMATION AND FILTERING FOR DOSE REDUCTION AND IMAGE QUALITY IN X-RAY IMAGING**

KONTROLLIERTE KOLLIMATION UND FILTERUNG ZUR DOSISREDUZIERUNG UND BILDQUALITÄT IN DER RÖNTGENBILDGEBUNG

COLLIMATION ET FILTRAGE CONTRÔLÉS POUR RÉDUCTION DE DOSE ET QUALITÉ D'IMAGE EN IMAGERIE PAR RAYONS X

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority: **12.12.2022 US 202263431770 P**

(43) Date of publication of application:
**22.10.2025 Bulletin 2025/43**

(73) Proprietor: **Koninklijke Philips N.V.
5656 AG Eindhoven (NL)**

(72) Inventors:
• **LEE, Brian Curtis**
**5656 AG Eindhoven (NL)**
• **SINHA, Ayushi**
**5656 AG Eindhoven (NL)**
• **SALEHI, Leili**
**5656 AG Eindhoven (NL)**
• **FOTOUHI, Javad**
**5656 AG Eindhoven (NL)**
• **FEIZPOUR, Amin**
**5656 AG Eindhoven (NL)**

(74) Representative: **Philips Intellectual Property &
Standards
High Tech Campus 52
5656 AG Eindhoven (NL)**

(56) References cited:
DE-B3- 102011 087 590     US-A1- 2015 272 531
US-A1- 2021 045 707     US-B2- 9 280 837

**Description**

<u>FIELD</u>

The following relates generally to the medical imaging arts, X-ray imaging arts (e.g., fluoroscopy arts, C-arm imaging arts, digital radiography arts, etc.), X-ray collimation arts, X-ray calibration arts, and related arts.

<u>BACKGROUND</u>

X-ray collimation is beneficial for improving X-ray image quality, for reducing the radiation dose to the patient, and for protecting radiation sensitive areas. Collimation can improve image quality by reducing scatter from the surrounding tissue, as well as reduce the likelihood of post-processing induced image over-exposure.

It is beneficial for an interventionalist to collimate closely around the area of interest during an X-ray guided procedure. The collimator typically includes X-ray opaque plates, and provides a rectangular and binary field of view, in which the X-ray signal is either 0% or 100% attenuated by the collimator (or filtered by a preset amount using a wedge filter).

However, this collimation is generally not optimal. For the first aim of reducing radiation dosage, the optimal field of view is rarely rectangular, due to the irregular and curved shape of most anatomical structures. Thus, a rectangular field of view exposes more tissue than is necessary to visualize the structure of interest, and potentially exposes the interventional staff to excess radiation as well.

Similarly, towards the second aim of improving image quality, undesirable X-ray scattering that can degrade the image can be produced by a scatter-inducing object embedded within a structure of interest (or an object inducing a large difference in intensity that may cause post-processing induced over-exposure). If the collimation is non-optimal such that a scatter-inducing object located outside of the structure of interest is nonetheless illuminated with X-rays, then this can also produce image-degrading scatter from the outside object into the image of the structure of interest.

Non-rectangular collimator technology has been described in the literature and introduced for non-fluoroscopic purposes in the market. For radiotherapy applications, non-rectangular collimators may use a number of mechanisms to achieve apertures of different shapes. Multi-leaf collimators (MLCs) are currently used in the radiation oncology field to restrict radiation to the tumor. Liquid collimators have been proposed using radiopaque fluid contained in malleable torus for finer control of the radiotherapy aperture contour. For example, DE102011087590 published June 6, 2013, and titled "Contour Collimator Having a Liquid Impermeable to X-rays" discloses a contour collimator for setting a contour of a radiation path of X-rays, the contour collimator forming an aperture and comprising a liquid impermeable to X-rays, in which the contour is formable by rotation of the liquid.

Although such collimators exist or have been proposed in the literature, no automatic control schemes for these systems have been developed, limiting their adoption into modern X-ray systems for fluoroscopy.

US 2015/272531 discloses a method for controlling a collimator using a scout image that includes a path formed from footprints of a medical device detected in a sequence of projection images during a exploration phase of progressing the device through a patient. US 9,280,837 discloses a method for automatically adjusting a collimator wedge position and/or aperture of a shutter mechanism using data, including the contour and size of a target structure or lesion, resulting from segmenting image data of a 3D representation of a patient's coronary artery tree's vessel segments in a region of interest associated with a precalculated optimal viewing angle. US 2021/045707 discloses a system that includes a pre-exposure device comprises a blocking component having an arrangement of openings across an extent of the component that is configured, when active, to reside within the path of an x-ray beam and a method of adjusting exposure of the x-ray imaging system using an image density map calculated from pre-exposure images acquired while the pre-exposure device is active.

The following discloses certain improvements to overcome these problems and others.

<u>SUMMARY</u>

The invention is defined in the independent claims. Specific embodiments thereof are defined in the dependent claims.

One advantage resides in providing for rapid subject-specific optimization of X-ray collimator settings of an X-ray imaging device.

Another advantage resides in providing collimation of an X-ray device using a non-rectangular X-ray collimator.

Another advantage resides in providing collimation of an X-ray device with a non-binary attenuation process.

Another advantage resides in calculating a confidence value for a collimation process for a collimator of an X-ray device.

Another advantage resides in providing a collimation to reduce scatter by collimating out a scatter-induced object.

Another advantage resides in providing a collimation to reduce radiation exposure to multiple and distinct regions of interest.

A given embodiment may provide none, one, two, more, or all of the foregoing advantages, and/or may provide other advantages as will become apparent to one of ordinary skill in the art upon reading and understanding the present disclosure.

## BRIEF DESCRIPTION OF THE DRAWINGS

The disclosure may take form in various components and arrangements of components, and in various steps and arrangements of steps. The drawings are only for purposes of illustrating the preferred embodiments and are not to be construed as limiting the disclosure.

FIGURE 1 diagrammatically illustrates an imaging device in accordance with the present disclosure.
FIGURE 2 diagrammatically illustrates an imaging device monitoring method using the imaging device of FIGURE 1.
FIGURE 3 diagrammatically shows an X-ray imaging system including subject-specific optimization of collimator settings of a non-polygonal X-ray beam collimator.
FIGURE 4 diagrammatically shows an X-ray imaging system including subject-specific optimization of collimator settings of a polygonal X-ray beam collimator.

## DETAILED DESCRIPTION

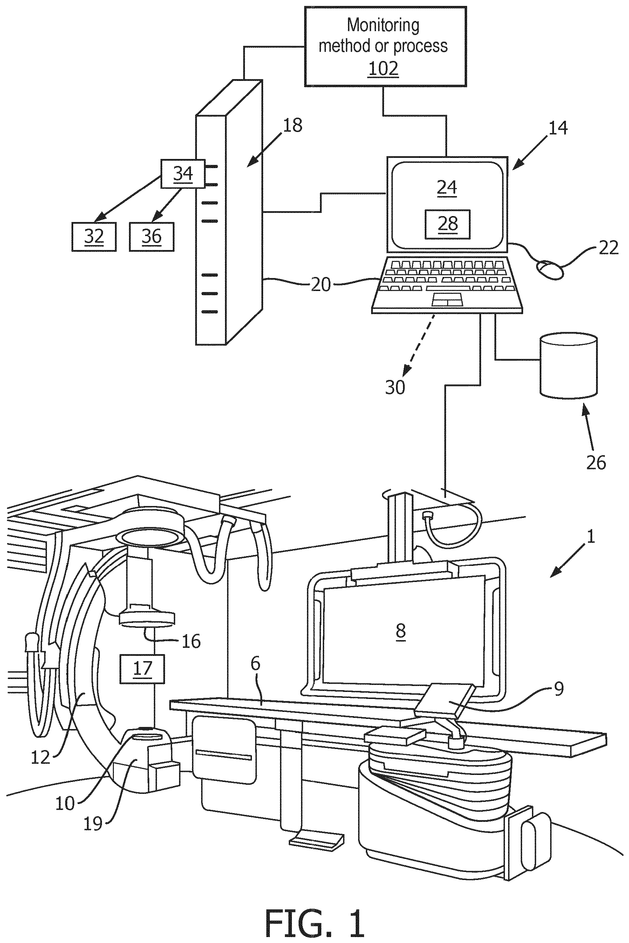

An illustrative imaging system **1** (e.g., medical imaging system) is shown in FIGURE 1. The imaging system **1** may be an image guided therapy (IGT) system that employs imaging to guide a medical procedure. In embodiments, the imaging system 1 may include one or more of an X-ray imaging device (also referred to as a fluoroscopic imaging device), a digital radiography (DR) imaging device, a computed tomography (CT) imaging device, or another imaging device that utilizes X-ray imaging (hereinafter referred to as an "X-ray imaging device" or variants thereof). In the embodiment shown in FIGURE 1, the imaging system **1** includes a C-arm X-ray imaging device **12** having a C-arm which may be used for image guided therapy (IGT). As shown in FIGURE 1, the X-ray imaging device may include one or more components, such as the diagrammatically shown X-ray collimator **10**. In the X-ray imaging system **1** in FIGURE 1, the X-ray imaging device **12** is in operative communication with a device controller **14** configured to control operation of the X-ray imaging system **1.**

The X-ray collimator **10** of FIGURE 1 is configured to collimate an X-ray beam of the X-ray imaging device **12.** In some embodiments, the X-ray collimator **10** is configured to provide an X-ray aperture having polygonal edges (e.g., at least two polygonal edges) that are neither perpendicular nor parallel to each other. In example embodiments, the collimator may include a set of mechanical shutters of different shapes that are able to translate and rotate independently to produce complex profiles including concave shapes. In example embodiments, the X-ray collimator **10** may include a shutter system in which each shutter is constructed of a thin, flexible material that is able to create curved profiles. In example embodiments, the X-ray collimator **10** may comprise an LCD array, where serial polarizers control the X-ray exposure on a pixel level and may provide partial attenuation of each part of the beam selectively. In example embodiments, the X-ray collimator **10** may comprise a liquid collimator including fluid bladders selectively fillable with a radiopaque fluid. In example embodiments, the X-ray collimator **10** may comprise a multi-leaf collimator (MLC). In embodiments, the X-ray collimator **10** may be configured to provide non-binary X-ray attenuation. These are merely illustrative example embodiments and should not be construed as limiting .

The X-ray imaging device **12** further includes an X-ray detector **16** that is configured to detect the X-ray radiation emitted by an X-ray source **19** (e.g.,,, an X-ray tube) after the X-rays pass through the collimator **10** and an examination region **17.** In the illustrative C-arm X-ray image system of FIGURE 1, the X-ray source **19** (e.g., comprising an X-ray tube) and the X-ray detector **16** are mounted on opposite ends of the C-arm. In other embodiments, the configuration may be different, e.g., in a DR the X-ray source may be mounted on a portable unit and the X-ray detector may be a separate flat-panel X-ray detector that may be disposed under a patient lying in a bed. For example, in a CT scanner embodiment, the X-ray tube and X-ray detector are mounted on a rotating gantry. These are merely illustrative embodiments that should not be construed as limiting.

In embodiments, the collimator **10** is positioned in front of the X-ray source **19** to collimate the X-ray beam output by the X-ray source **19** before the X-ray beam impinges on a patient or other subject disposed in the examination region **17.** During operation to acquire imaging data of a patient or other imaging subject (not shown), that imaging subject is disposed in the examination region **17.** The detector **16** may comprise a detector array as shown in FIGURE 1, and the X-ray tube **19** may emit a cone beam of X-rays that pass through the collimator **10,** and then the examination region **17** and thence impinge on the detector array **16.**

In FIGURE 1, the detector **16** is also in electronic communication with the device controller **14,** such as a workstation computer, or more generally a computer. Images produced by the X-ray imaging device **12** via the X-ray radiation generated by the X-ray tube **19** are processed by the device controller **14** and/or another electronic processing device, which may for example be implemented as a server computer **18** as shown. The illustrative X-ray imaging device **12** employs a C-arm configuration used in X-ray imaging systems for cardiac imaging, image-guided therapy (IGT), or other clinical applications. The X-ray tube **19,** the collimator **10,** and the detector **16** of the C-arm X-ray imaging device **12** may be moved on robotic arms or the like to different vantage points (called "views") around the patient to, for example, provide clinically significant views of the heart, or, for example, in an IGT to provide a chosen view of an interventional procedure. As a nonlimiting example, the illustrative C-arm X-ray imaging device **12** could be a Philips Azurion IGT system (available from Koninklijke Philips N.V., Eindhoven, the Netherlands). The X-ray imaging device **12,** as shown in FIGURE 1, further includes a patient support 6 and a large display **8** for presenting images for guiding an operator in performing an interventional procedure or other image-guided therapy related operation.

The processing device **18** of FIGURE 1 may include a workstation, a server computer (as shown), or a plurality of server computers, e.g., interconnected to form a server cluster, cloud computing resource, various combinations thereof, or so forth, to perform complex computational tasks. In the illustrative embodiment, the device controller **14** may be integrated into an on-board computer of the display system **8,** an additional computing component **9,** etc. In example configuration, the device controller **14** may be provided for controlling the X-ray imaging device **12** to perform image acquisition. The device controller **14** and/or server **18** of FIGURE 1 includes components, such as a hardware processor **20** (e.g., a microprocessor), at least one user input device (e.g., a mouse, a keyboard, a trackball, and/or the like) **22,** and a display device 24 (e.g., an LCD display, plasma display, cathode ray tube display, and/or so forth).

The hardware processor **20** (also referred to a "processor") is operatively connected with one or more non-transitory storage media **26**. The non-transitory storage media **26** may, by way of non-limiting illustrative example, include one or more of a magnetic disk, RAID, or other magnetic storage medium; a solid-state drive, flash drive, electronically erasable read-only memory (EEROM) or other electronic memory; an optical disk or other optical storage; various combinations thereof; or so forth; and may be for example a network storage, an internal hard drive of the workstation **18,** various combinations thereof, or so forth. It is to be understood that any reference to a non-transitory medium or media **26** herein is to be broadly construed as encompassing a single medium or multiple media of the same or different types. Likewise, the processor **20** may be embodied as a single hardware processor or as two or more hardware processors. The non-transitory storage media **26** stores instructions executable by the at least one processor **20.** The instructions may include instructions to generate a visualization of a graphical user interface (GUI) **28** for display on the display device **24** (and/or elsewhere, for example on the IGT display **8** and/or auxiliary display **9).**

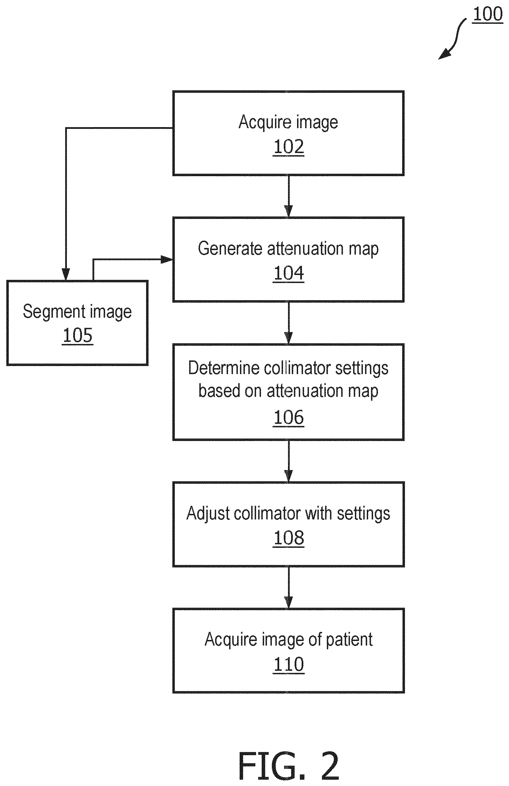

With reference to FIGURE 2, and with continuing reference to FIGURE 1, an illustrative method **100** is diagrammatically shown as a flowchart. To begin the method **100,** a patient is loaded into the examination zone **17.** At an operation **102,** the controller **14** controls the X-ray imaging device 1 to acquire an initial X-ray image of the patient in the examination zone **17.** To acquire the X-ray image, X-rays are emitted by the X-ray tube **19** pass through the collimator **10,** and then through the examination region **17,** and thence are detected by the X-ray detector array **16.** The device controller **14** and/or the server **18** is configured to receive the X-ray image of the patient in the examination zone **17.** In some embodiments, this initial image acquired at the operation **102** may be captured as a scout image or the like, and is generally acquired with no collimation or with the collimator **10** set to a wide beam that is expected to encompass all structure(s) of interest for the imaging session. In some embodiments, the image acquired at operation **102** may be acquired at a low X-ray beam intensity to limit radiation exposure of the patient.

At operation **104,** the controller **14** generates an X-ray beam attenuation map **32** having pixels representing target X-ray beam attenuation values for use in imaging the patient with the X-ray imaging device **12.** To do so, at operation **105,** the controller optionally segments the image acquired at the operation **102** by analyzing or processing the image to identify and/or delineate image features indicative of X-ray exposure characteristics of the subject (e.g., location of types of tissue of the subject, location of structures within the subject, an implant with the subject, other spatial information of the imaged portion of the subject, etc.) in the image. The controller uses such image features to determine regions of the subject for X-ray exposure during imaging and/or for which X-ray exposure is to be minimized or avoided entirely. For example, if the imaging is for image guidance during a cardiology procedure, then the image features may be used to determine the structures of interest for X-ray exposure during imaging may include the heart and coronary arteries, while avoiding X-ray exposure to an implanted pacemaker or other structure likely to produce substantial X-ray scattering. In some embodiments, the structure(s) of interest may be manually delineated in the image (e.g., by the controller **14** interaction with a user), or may be automatically delineated by automatic segmentation by the controller **14** (e.g., known image processing and/or feature identification techniques), or a combination thereof. The controller **14,** at operation **104,** then constructs the attenuation map **32** defining optimal X-ray imaging exposure for regions (e.g., structures) of the patient when imaging the patient during a procedure. In embodiments, the attenuation map defines region(s) of low or zero attenuation, where X-ray imaging should be not be applied (collimated), and high attenuation regions, where X-ray imaging should be applied to image the structures.

In some embodiments, the X-ray beam attenuation map **32** is defined on a pixel-by-pixel level, where a scalar value at each pixel indicates the desired amount of attenuation at this point in the imaging field.

In some embodiments, the X-ray beam attenuation map **32** is generated by a method that scores or otherwise calculates a value of the optimal attenuation levels at different regions of the imaged portion of the subject. The method may extract the image features in the image by an image processing feature identification technique and/or a segmentation technique, etc., and then obtain scatter data related to the identified features and the scatter data may be score or otherwise valued according at attenuation levels. In some embodiments, method may further obtain other scoring data related to the identified region, such as sensitivity of the region to X-ray exposure, relevant to a particular procedure, etc.. The method may calculate attenuation levels for each different region based on the scored scatter data and/or scored other data obtained for the identified features in or near the different region. In some embodiments, the scored scatter data and/or scored other data may be scored image data valued on a pixel basis and compared to the identified feature in the imaged portion to provide a pixel-by-pixel calculation of the attenuation levels for a region.

In some embodiments, the X-ray beam attenuation map **32** is generated by a computational model **34** or algorithm that computes the optimal attenuation levels at different regions of the image by analyzing the X-ray image according to spatial information, anatomical context, and procedural context. The analysis may include determining scatter values for the different regions.

In some embodiments, the computational model **34** that generates the X-ray beam attenuation map 32 may be a set of linear operators acting on the X-ray image intensities.

In some embodiments, the computational model that generates the X-ray beam attenuation map 32 may be a set of pre-defined computer vision filters, such as convolutional filters like a vessel enhancing filter or an edge enhancing filter.

In some embodiments, the computational model **34** may be a machine learning model based on hand-crafted feature extractors.

In some embodiments, the X-ray beam attenuation map **32** is generated by the computational model or algorithm comprising an artificial neural network (ANN) **34** implemented in the server **18.** Inputs are provided to the ANN **34** to generate the X-ray beam attenuation map **32.** The inputs to the ANN **34** may include, for example, the X-ray image and/or image features extracted from the X-ray image that are indicative of the X-ray exposure characteristics of the subject (e.g., location of types of tissue of the subject, location and/or condition of particular anatomical structures of the subject, location of an implant within the subject, etc.). In embodiments, the inputs may include further optional inputs related to spatial information such as at least one of a region of the subject to be imaged and/or a region of the subject to be excluded from exposure to the X-ray beam, as well as optional input information describing the context of the medical procedure. In some embodiments, the information input to the ANN **34** further includes an X-ray scatter model representing X-ray scatter from tissue and/or an implant within the subject, as well as current x-ray system settings influencing scatter such as tube voltage, current, etc. At an optional operation **105,** automatic segmentation of the acquired X-ray image may be performed to determine the spatial information and/or the image features. In order to produce the attenuation map 32 from a combination of some of the above inputs, the ANN 34 may be trained to receive as input and analyze, at a minimum, the X-ray image and optionally other inputs. The computation could be a simple concatenation of numerical representations of the input for an encoder-decoder-like ANN, or could take the form of additional transformer tokens in the case of a transformer-like ANN, among other options. The ANN 34 may produce, as output, an image whose pixel values correspond to the desired target attenuation values for collimation. In some embodiments, the output image is a constructed attenuation map 32 on a pixel level. In some embodiments, the output image is delineated by regions of interest (for instance, anatomical structures or surgical devices) and each region of interest has an associated attenuation value.

In some embodiments, the output of the ANN **34** is the desired attenuation value to be achieved by collimation. In other example embodiments, the output of the ANN **34** is an array of intermediate values that is mapped to the desired attenuation value by a function, where the function may be a lookup table or a linear mapping or otherwise.

In some embodiments, in response to the inputs, the ANN **34** may also generate an X-ray beam attenuation confidence map **36** having pixels representing confidence values for the X-ray beam attenuation values of corresponding pixels of the X-ray beam attenuation map **32.**

In some embodiments, the ANN **34** may also directly regress the set of collimator parameters (for instance, four positional parameters for a centered rectangular collimator). In an example where the collimator **10** is a four-leaf rectangular collimator, instead of an encoder-decoder architecture producing an intermediate pixel-level or region-of-interest-level attenuation map on which the collimator settings are determined, the ANN **34** may perform a linear fit from encoded features to a set of four collimator parameters, where each parameter is associated to the displacement of each of four collimator shutters from a default position.

The ANN **34** may be trained prior to generating the X-ray beam attenuation map **32** and the confidence map **36.** To do so, in embodiments, previously acquired X-ray images with regions of interest (ROIs) are input to the ANN **34.** The importance of showing or collimating each ROI is scored, manually or automatically, based on the content of the image and the context of the medical procedure. For example, the previously acquired X-ray images may be manually labeled with scores according to the content of the image and procedure. For another example, a computer process or algorithm (such as included in the ANN) may identify features in the image and determine labeling or other indication of scores to portions of the image indicating importance of collimating those portions based on the identified features and provided context of the medical procedure. In some embodiments, the labeling and scoring is on a pixel-by-pixel basis.

In an example, the ANN **34** is being trained for X-ray guided orthopedic procedures in which visualizing the bones is important, but visualizing soft tissue is not important. In this example, the bones involved in the procedure are scored with the highest score, and skin, fat, muscle, and air are given a low score. However, observation of the neighborhood of the surgical site may be important to prevent adverse side effects of the surgical procedure, so soft tissue closer to the surgical site is instead scored moderately high. Bony structures not relevant to the procedure would be given a low score. Although vasculature is not visible in X-ray images without contrast, a priori knowledge of the typical anatomy may be used to estimate location of important arteries and veins that could be damaged during the procedure and these locations may be given a high score to ensure visibility. Radiation-sensitive organs like the colon may be given the lowest score to ensure that they are collimated. A metal implant in the neighborhood of the surgical site would be known to cause some degree of scattering and may be given a low score depending on proximity to important ROIs. In embodiments, such scoring is performed for a dataset of images and input to train the ANN **34** to predict the optimal attenuation map from an intraprocedural image. In some embodiments, the scoring is performed on a pixel-wise level and without specific ROIs. These operations may be repeated until a stopping criterion is met. In an example, the stopping criterion is met when the value of the objective function used to optimize the ANN **36** converges on an independent dataset.

In some embodiments, the ANN **34** is trained in a semi-supervised or an unsupervised manner without manually labeled ground truth attenuation maps. The desired attenuation may be extracted from an intermediate algorithm such as one of the pre-defined algorithms described above or from such an input synthesized with a partially labeled dataset.

At an operation **106,** X-ray collimator settings are determined for the X-ray collimator **10** to implement the X-ray beam attenuation map **32.** In some embodiments, the determination operation **106** includes adjusting the determined X-ray collimator settings based on the X-ray beam attenuation confidence map **36.** Put another way, the operation **106** transforms the attenuation map generated in the operation **104** into a set of physically realizable collimator setting values (e.g., shutter positioning/orientation) that configure the collimator **10** to define an aperture for the X-ray beam output by the X-ray source **19** that shapes the collimated X-ray beam to conform with the attenuation map. In an example where the collimator **10** is a set of four shutters oriented to create a rectangular aperture, the position of the four positional parameters of the shutters may be optimally selected to simultaneously minimize radiation to the patient's body, while maximizing the amount of important features visible in the rectangular aperture. In another example, where the collimator **10** has pixel-level control of attenuation levels, the collimator **10** is set to match the produced attenuation map **32** when confidence is high, and attenuation is reduced in areas where confidence is low.

In one example, when the collimator **10** comprises an MLC, the determined X-ray collimator settings are collimator leaf settings of the MLC. In another example, when the collimator **10** provides non-binary X-ray attenuation, the generated X-ray beam attenuation map **32** has non-binary pixel values, and the determined X-ray collimator settings provide partial X-ray attenuation in correspondence with the non-binary pixel values of the X-ray beam attenuation map **32.**

At an operation **108,** the X-ray collimator **10** is adjusted to the determined X-ray collimator settings.

At an operation **110,** after the adjusting, at least one clinical image of the subject in the examination region **17** is acquired. Optionally, the operations **104-108** are repeated given the newly acquired clinical image in order to set collimation for the next image.

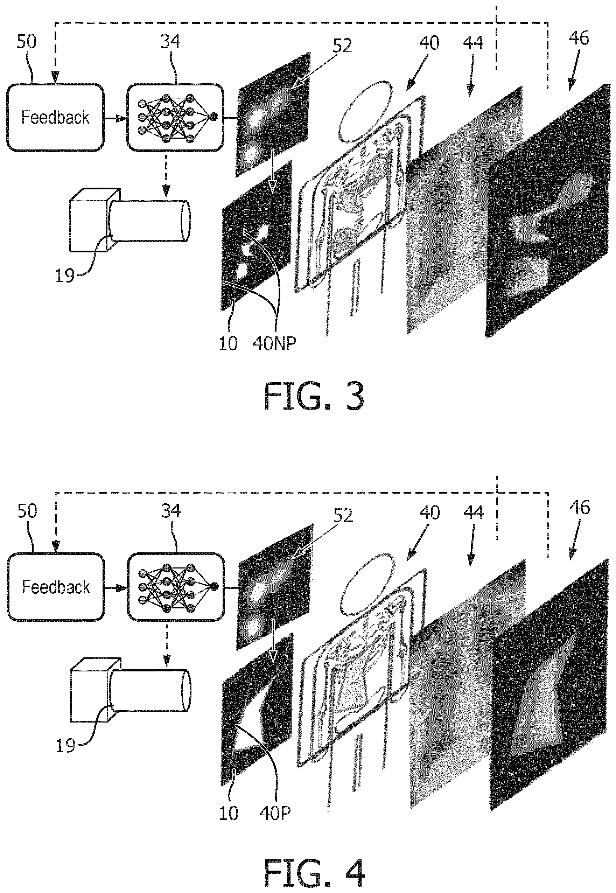

With reference now to FIGURES 3 and 4, method examples based on the method 100 of FIGURE 2 are presented in diagrammatic fashion. Each of FIGURES 3 and 4 illustrate the X-ray source 19 and the configured collimator 10 disposed in front of the X-ray source 19. The example of FIGURE 3 employs a non-polygonal X-ray beam collimator 10; that is, the collimator 10 of FIGURE 3 is capable of being configured by its settings to form an X-ray aperture **40NP** whose edges are not straight lines. By contrast, the example of FIGURE 4 employs a polygonal X-ray beam collimator 10; that is, the collimator 10 of FIGURE 4 is configurable by its settings to form an X-ray aperture 40P whose edges are exclusively straight lines. For example, the non-polygonal collimator 10 of FIGURE 3 could be implemented as a liquid collimator including fluid bladders selectively fillable with a radiopaque fluid to provide the X-ray aperture **40NP** with edges that are not straight lines defined by perimeters of the filled bladders. In another example, the polygonal collimator **10** of FIGURE 4 could be implemented as a set of radiopaque plates having straight edges defining the straight edges of the polygonal X-ray aperture **40P,** where the plates can be moved both translationally and rotationally to form the polygonal X-ray aperture **40P** having polygonal edges including at least two polygonal edges that are neither perpendicular nor parallel to each other.

In each of FIGURES 3 and 4, a patient **40** is also diagrammatically shown, with the area of the respective X-ray aperture **40NP** or **40P** diagrammatically indicated by a shaded area of the patient **40.** Also shown in each of FIGURES 3 and 4 is an initial (e.g., scout) image **44,** for example acquired in the operation **102** of FIGURE 2. This initial scout image **44** is suitably initially acquired with the collimator **10** removed (or, alternatively, with the collimator **10** in place but with the collimator settings being set to provide a sufficiently wide-open aperture to ensure the aperture encompasses the structure(s) of interest). This initial image **44** provides initial feedback **50** for the aperture settings adjustment. The feedback **50** may include other spatial information such as identification or delineation of the structure(s) of interest in the image **44** or other image features indicative of X-ray exposure characteristics of the subject. This feedback is input to the ANN **34** which processes the inputs **50** to produce the attenuation map **52** (e.g., as corresponding to operation **104** of FIGURE 2). Per the operation **106** of FIGURE 2, the attenuation map **52** is then converted to physically realizable collimator settings of the collimator **10** thereby producing the configured collimator **10** shown in each of FIGURES 3 and 4.

In some embodiments, this process may be repeated during an examination to accommodate changes in the imaging setup. For example, during a cardiac IGT procedure the operator may move the C-arm **12** to acquire a new view, at which point the feedback **50** becomes the image **46** from the last view which is then adjusted by the ANN **34** to update the attenuation map 52 and the configuration of the collimator **10.** Thus, the collimator settings may be adjusted in real-time over the course of the procedure.

## EXAMPLE

The following describes some illustrative examples of the imaging system in more detail. In one example, the model **34** (e.g., the ANN) receives several inputs including but not limited to an acquired X-ray image, prior knowledge of the anatomy to be imaged/targeted, the procedure phase/type, radiation sensitive areas to avoid, the expected level of scatter from each object in the image, etc. The model 34 outputs the optimal pixel-wise filtration/collimation level in a heatmap or other dense data type. A control scheme changes the collimation configuration to best match the optimal collimation output within the mechanical/physical bounds of the imaging system.

The X-ray beam attenuation map 32 comprises a heatmap indicating the desired attenuation of each part of the X-ray beam. The heatmap may be calculated based on the importance of visualizing each part of the anatomy, the expected level of noise produced by each object that the X-ray beam passes through, particularly radiation sensitive parts of the anatomy such as the pelvic area of a pregnant patient, and so on.

As an example, a simple encoder-decoder neural network architecture, such as in one embodiment of the model **34,** may be trained to produce this output. The model **34** may receive as input a combination of the current X-ray image, prior information about the patient anatomy or procedure (such as the anatomical structure to be imaged and areas to protect), and noise models or scatter models indicative of the amount of scatter produced by materials/objects detected in the image. Methods for estimation of X-ray scatter are well described in the field and may be based on factors, such as material type, shape, and orientation relative to the anatomy. In some embodiments, a segmentation or object detection algorithm may be applied to identify objects of interest in the image which influence the above factors.

The X-ray beam attenuation map 32 may then be passed to the collimation software, which would attempt to match collimation settings to the filtration/collimation levels represented by the values on the X-ray beam attenuation map 32 as closely as possible within physical limitations. The produced image may be then be fed back into the model 34 in a real-time loop.

In another embodiment, a simpler polygonal collimator 10 capable of creating both concave and convex apertures can be used to try to match to the X-ray beam attenuation map 32. This collimator 10 could be a set of leaves with hinges, or leaves that do not span the full extent of the image.

The training data for the model **34** could be generated by annotating previously acquired X-ray images with regions of interest and quantitatively scoring the importance of either showing or collimating each region (as described above). Scoring could indicate the likelihood of scattering from a particular region of interest, or the importance of showing a particular part of the anatomy during the given phase of the procedure, or the importance of protecting the region of interest from excessive radiation, or a combination of these factors.

The control scheme for the collimator **10** would receive the X-ray beam attenuation map 32 as an input and produce the optimal collimator configuration given the physical limitations of the collimator **10.** This could be performed by a high-dimensional optimization of the collimator settings with the objective of matching the resulting filtration/collimation-per-detector-pixel to the desired X-ray beam attenuation map **32.**

For instance, for a rectangular collimator **10,** this optimization could be a minimization with respect to a displacement value associated to each shutter according to Equation (1):

$$argmin_{x_1, x_2, x_3, x_4} \Sigma [H(\cdot) - f(x_1, x_2, x_3, x_4, \cdot)]^2 \qquad (1)$$

where *H* is the X-ray beam attenuation map **32** and *f* is a function describing the resulting collimation/filtration per pixel based on the collimation parameters $x_1$, $x_2$, $x_3$, $x_4$, where this describes a shutter configuration where the left shutter is extended to the right by $x_1$, the right shutter is extended to the left by $x_2$, and so on. This produces an image of the same dimensions as H, where in the case of binary collimation, the areas associated to the exposed areas of the aperture have attenuation value 0% and the occluded areas have attenuation value 100%.

Equation 2 is a generalized version of Equation 1 for a complex collimator **10:**

$$argmin_\alpha \ \Sigma[H(\cdot) - f(\alpha, \cdot)]^2 \qquad (2)$$

where $\alpha$ is a pixel-wise attenuation value from 0% to 100% describing amount of attenuation of the X-ray beam at this particular pixel prior to exposure to the tissue, where 100% would be equivalent to collimation with a lead shutter. This or the above optimization could be performed using the model **36.** The imaging system could continuously update the collimation settings in real time as new images arrive from the detector **16.**

In another example, a blocked area in the center of the collimator **10** may be surrounded by open areas. Non-binary attenuation may then be performed using a filter, rather than a collimator. The collimation could be used to reduce scattering or otherwise improve image quality. To do so, the X-ray beam attenuation map **32** may be generated dynamically using a pixel-by-pixel (i.e., 0-100%) attenuation setting that is updated frame-by-frame as a contrast agent or contrast region is added, a field of view (FOV) of the imaging device **1** is moved, and so forth. To do so, an image is acquired, an X-ray beam is shuttered, the collimator settings are adjusted, the X-ray beam is unshuttered, and another image is acquired. This process is prepared to optimize collimation. In another example, for a small FOV, the collimation is adjusted to fully open the collimator **10** to take an image, after which the small FOV is reset to accommodate motion. Using partial attenuation around a margin of the FOV may provide enough data to dynamically adjust collimation without an extra collimator expansion step.

In some embodiments, the optimal pixel-wise filtration/collimation level is accompanied by a confidence estimation. The confidence estimate may be additionally input into the control scheme that changes the collimation configuration to best match the optimal collimation output within the mechanical/physical bounds of the system and may help set the aggressiveness of the collimation configuration. For instance, where confidence is high, collimation configuration may be tightly bound to the boundaries of the regions to be collimated. However, where confidence is low, collimation configuration may leave some room for error around the regions to be collimated. There may be a bias toward revealing more around a margin of the FOV (i.e., for looser collimation) if the confidence is low. The margin may be partially attenuated (i.e., non-binary) or opened slightly more to reveal the margin (i.e., binary).

In some embodiments, users may additionally specify (e.g., using the GUI **28)** where collimation configurations may need to be changed. Users may, for instance, draw on the resulting images using different options (green to include, red to exclude) to include or exclude regions in the collimation configuration.

In one particular example, the imaging system may be used for a pregnant patient with hip fracture due to motor vehicle accident. For trauma patients, X-ray is the imaging modality of choice due to its speed and the emergent nature of the patient/setting. However, an unborn fetus is particularly susceptible to radiation, with severe consequences (i.e., cancer, growth restrictions, brain dysfunction, etc.) if doses exceed 0.5 Gy, a level which is not necessarily dangerous to adults. Although diagnostic X-rays tend not to be a concern for a fetus, interventional X-ray may reach dangerous levels. Modern collimators do not have the ability to exclude objects from the center of the field of view, they are mainly designed to reveal objects by opening an aperture for the X-ray beam. As such, lead shielding and beam positioning are the only way to protect pregnant patients who need emergent X-ray in the pelvic region. This invention would enable substantially increased precision in shielding non-rectangularly shaped objects that are sensitive to radiation, such as a fetus, by automatically detecting the sensitive area and controlling the collimator configuration to constantly block radiation to this region regardless of the gantry position.

In another particular example, tissue surrounding a deep vein thrombosis (DVT) treatment area may be highly sensitive to radiation. Radiation is known to cause blood clots that may result in dangerous embolism. However, due to the sprawling branch-like structure of the vasculature, it may be difficult to visualize a part of the vessels while excluding all else around it with a rectangular collimator. This collimator settings could open an aperture in the shape of a blood vessel based on the output of the model **34** or a vessel segmentation algorithm to prevent further clot formation.

The disclosure has been described with reference to the preferred embodiments. Modifications and alterations may occur to others upon reading and understanding the preceding detailed description. It is intended that the exemplary embodiment be construed as including all such modifications and alterations insofar as they come within the scope of the appended claims or the equivalents thereof.

## Claims

1.    A system **(1)** for providing X-ray collimator settings, the system **(1)** comprising:
     a controller **(14)** comprising a processor in communication with memory, the processor configured to:

     receive an X-ray image of a subject from an X-ray imaging device, wherein the X-ray imaging device **(12)** includes an X-ray collimator **(10)** configured to collimate an X-ray beam of the X-ray imaging device for imaging the subject;
     identify, in the X-ray image, at least one image feature indicative of at least one X-ray exposure characteristic of the subject;

determine, in the X-ray image, the structures of interest for X-ray exposure during imaging and the structures likely to produce substantial X-ray scattering to avoid during imaging;

generate an X-ray beam attenuation map (32) defining an X-ray imaging exposure for the determined structures of the subject when imaging the patient during a procedure; and

determine at least one X-ray collimator setting for the X-ray collimator based on the X-ray beam attenuation map.

2. The system (1) of claim 1, wherein the X-ray beam attenuation map (32) defines the one or more target X-ray beam attenuation values to correspond to one or more pixels representing the one or more regions of the subject.

3. The system (1) of claim 1, wherein the at least one X-ray exposure characteristic of the subject comprises at least one of: (i) location of a type of tissue of the subject, (ii) location of an anatomical structure within the subject, (iii) spatial information of anatomy of the subject, or (iv) location of an implant within the subject.

4. The system (1) of claim 1, wherein the processor is further configured to:

obtain an X-ray scatter model representing at least one of (i) X-ray scatter from at least one of tissue or anatomical structures of the subject, (ii) X-ray scatter from an implant of the subject, (iii) spatial information related to the at least one region of the subject, or (iv) procedural context information; and

generate the X-ray beam attenuation map (32) by application of a computational model (34) trained to generate the X-ray beam attenuation map based on the X-ray scatter model and the at least one image feature.

5. The system (1) of claim 4:

wherein the computational model is further trained to generate a confidence map (36) defining one or more confidence value for the one ore more X-ray beam attenuation values of the X-ray beam attenuation map; and

wherein the processor is further configured to determine the at least one X-ray collimator setting based on the X-ray beam attenuation confidence map.

6. The system (1) of claim 5, wherein:

the X-ray beam attenuation map (32) defines the one or more target X-ray beam attenuation values to correspond to one or more pixels representing the one or more regions of the subject; and

the confidence map (32) defines the one or more confidence values to correspond to the one or more pixels representing the one or more regions of the subject.

7. The system (1) of claim 1, wherein the processor is further configured to:

perform automatic segmentation of the X-ray image to identify the at least one image feature.

8. The system (1) of claim 1, wherein the processor is further configured to:

adjust the X-ray collimator (10) to the at least one X-ray collimator setting.

9. The system (1) of claim 1, wherein the processor is configured to:

generate the X-ray beam attenuation map (32) to include one or more non-binary pixel values; and

determine the at least one X-ray collimator setting to provide partial X-ray attenuation in correspondence with the non-binary pixel values of the X-ray beam attenuation map (32).

10. The system (1) of claim 4, further comprising a second processor configured to train the computational model, the second processor configured to:

receive previously acquired X-ray images from at least one previous procedure;

annotate regions of interest (ROIs) within the previously acquired X-ray images;

assign a score indicating importance of showing or collimating each annotated ROI; and

generate the computational model based on the annotated ROI and assigned score.

11. The system (1) of claim 1, further comprising the X-ray imaging device (12) including the X-ray collimator (10).

12. The system **(1)** of claim 11, wherein the X-ray collimator **(10)** comprises a multi-leaf collimator (MLC), and the at least one X-ray collimator setting is at least one collimator leaf setting of the MLC.

13. A method of providing X-ray collimator settings, the method comprising:

receiving an X-ray image of a subject from an X-ray imaging device, wherein the X-ray imaging device **(12)** includes an X-ray collimator **(10)** configured to collimate an X-ray beam of the X-ray imaging device for imaging the subject;

identifying, in the X-ray image, at least one image feature indicative of at least one X-ray exposure characteristic of the subject;

determining, in the X-ray image, the structures of interest for X-ray exposure during imaging and the structures likely to produce substantial X-ray scattering to avoid during imaging;

generating an X-ray beam attenuation map **(32)** defining an X-ray imaging exposure for the determined structures of the subject when imaging the patient during a procedure; ,and

determining at least one X-ray collimator setting for the X-ray collimator based on the X-ray beam attenuation map.

14. The method of claim 13, further comprising:

obtaining an X-ray scatter model representing at least one of (i) X-ray scatter from at least one of tissue or anatomical structures of the subject, (ii) X-ray scatter from an implant of the subject, (iii) spatial information related to the at least one region of the subject, or (iv) procedural context information; and

generating the X-ray beam attenuation map **(32)** by application of a computational model **(34)** trained to generate the X-ray beam attenuation map based on the X-ray scatter model and the at least one image feature.

15. A non-transitory computer-readable storage medium having stored a computer program comprising instructions, which, when executed by a processor, causes the processor to operate the method of claim 13 or 14.

**Patentansprüche**

1. System **(1)** zum Bereitstellen von Röntgenkollimatoreinstellungen, das System **(1)** umfassend:
eine Steuereinheit **(14),** die einen Prozessor in Kommunikation mit Speicher umfasst, wobei der Prozessor konfiguriert ist zum:

Empfangen eines Röntgenbildes eines Subjekts von einer Röntgenbildgebungsvorrichtung, wobei die Röntgenbildgebungsvorrichtung **(12)** einen Röntgenkollimator (10) beinhaltet, der konfiguriert ist, um einen Röntgenstrahl der Röntgenbildgebungsvorrichtung zur Bildgebung des Subjekts zu kollimieren;

Identifizieren mindestens eines Bildmerkmals, das für mindestens eine Röntgen-Aussetzungseigenschaft indikativ ist, im Röntgenbild;

Bestimmen der Strukturen von Interesse für eine Röntgen-Aussetzung im Röntgenbild während einer Bildgebung und der Strukturen, die wahrscheinlich eine wesentliche Röntgenstreuung erzeugen, die während der Bildgebung zu vermeiden sind;

Erzeugen einer Röntgenstrahl-Abschwächungskarte (32), die eine Röntgenbildgebungsaussetzung für die bestimmten Strukturen des Subjekts bei der Bildgebung des Patienten während einer Prozedur definiert; und

Bestimmen mindestens einer Röntgenkollimator-Einstellung für den Röntgenkollimator basierend auf der Röntgenstrahl-Abschwächungskarte.

2. System (1) nach Anspruch 1, wobei die Röntgenstrahl-Abschwächungskarte (32) den einen oder die mehreren Ziel-Röntgenstrahl-Abschwächungswerte definiert, um einem oder mehreren Pixeln zu entsprechen, die die eine oder mehrere Bereiche des Subjekts darstellen.

3. System **(1)** nach Anspruch 1, wobei die mindestens eine Röntgen-Aussetzungseigenschaft des Subjekts mindestens eines umfasst von: (i) Stelle eines Gewebetyps des Subjekts, (ii) Stelle einer anatomischen Struktur innerhalb des Subjekts, (iii) räumlicher Informationen über die Anatomie des Subjekts oder (iv) Stelle eines Implantats innerhalb des Subjekts.

4. System (1) nach Anspruch 1, wobei der Prozessor weiter konfiguriert ist zum:

Erhalten eines Röntgenstreumodells, das mindestens eines von (i) Röntgenstreuung von mindestens einem Gewebe oder anatomischen Strukturen des Subjekts, (ii) Röntgenstreuung von einem Implantat des Subjekts, (iii) räumlichen Informationen in Bezug auf mindestens einen Bereich des Subjekts oder (iv) Informationen zum Verfahrenskontext darstellt; und

Erzeugen der Röntgenstrahl-Abschwächungskarte (32) durch Anwenden eines Rechenmodells (34), das trainiert ist, um die Röntgenstrahl-Abschwächungskarte basierend auf dem Röntgenstreumodell und mindestens einem Bildmerkmal zu erzeugen.

5. System (1) nach Anspruch 4:

wobei das Rechenmodell weiter trainiert wird, um eine Konfidenzkarte (36) zu erzeugen, die einen oder mehrere Konfidenzwerte für den einen oder die mehreren Röntgenstrahl-Abschwächungswerte der Röntgenstrahl-Abschwächungskarte definiert; und

wobei der Prozessor weiter konfiguriert ist, um die mindestens eine Röntgenkollimator-Einstellung basierend auf der Röntgenstrahlabschwächungs-Konfidenzkarte zu bestimmen.

6. System (1) nach Anspruch 5, wobei:

die Röntgenstrahl-Abschwächungskarte (32) den einen oder die mehreren Ziel-Röntgenstrahl-Abschwächungswerte definiert, um einem oder mehreren Pixeln zu entsprechen, die die eine oder mehreren Bereiche des Subjekts darstellen; und

die Konfidenzkarte (32) den einen oder die mehreren Konfidenzwerte definiert, um dem einen oder den mehreren Pixeln zu entsprechen, die die eine oder mehrere Bereiche des Subjekts darstellen.

7. System (1) nach Anspruch 1, wobei der Prozessor weiter konfiguriert ist zum:
Durchführen einer automatischen Segmentierung des Röntgenbildes, um mindestens ein Bildmerkmal zu identifizieren.

8. System (1) nach Anspruch 1, wobei der Prozessor weiter konfiguriert ist zum:
Anpassen des Röntgenkollimators (10) auf die mindestens eine Röntgenkollimatoreinstellung.

9. System (1) nach Anspruch 1, wobei der Prozessor konfiguriert ist zum:

Erzeugen der Röntgenstrahl-Abschwächungskarte (32), um einen oder mehrere nicht-binäre Pixelwerte zu beinhalten; und

Bestimmen der mindestens einen Röntgenkollimator-Einstellung, um eine partielle Röntgenabschwächung in Übereinstimmung mit den nicht-binären Pixelwerten der Röntgenstrahl-Abschwächungskarte (32) bereitzustellen.

10. System (1) nach Anspruch 4, weiter umfassend einen zweiten Prozessor , der konfiguriert ist, um das Rechenmodell zu trainieren, wobei der zweite Prozessor konfiguriert ist zum:

Empfangen von zuvor erfassten Röntgenbildern aus mindestens einem vorherigen Verfahren; Kommentieren von Bereichen von Interesse (ROIs) innerhalb der zuvor erfassten Röntgenbilder;

Zuweisen einer Punktzahl, die die Bedeutung des Zeigens oder Kollimierens indiziert, jedem kommentierten ROI; und

Erzeugen des Rechenmodells basierend auf dem kommentierten ROI und der zugewiesenen Punktzahl.

11. System (1) nach Anspruch 1, weiter umfassend die Röntgenbildgebungsvorrichtung (12), die den Röntgenkollimator (10) beinhaltet.

12. System (1) nach Anspruch 11, wobei der Röntgenkollimator (10) einen Mehrblattkollimator (MLC) umfasst und die mindestens eine Röntgenkollimator-Einstellung mindestens eine Blatteinstellung des MLC ist.

13. Verfahren zum Bereitstellen von Röntgenkollimator-Einstellungen, wobei das Verfahren umfasst:

Empfangen eines Röntgenbildes eines Subjekts von einer Röntgenbildgebungsvorrichtung, wobei die Röntgenbildgebungsvorrichtung **(12)** einen Röntgenkollimator (10) beinhaltet, der konfiguriert ist, um einen Röntgenstrahl der Röntgenbildgebungsvorrichtung zur Bildgebung des Subjekts zu kollimieren;

Identifizieren mindestens eines Bildmerkmals, das für mindestens eine Röntgen-Aussetzungseigenschaft indikativ ist, im Röntgenbild;

Bestimmen der Strukturen von Interesse für eine Röntgen-Aussetzung im Röntgenbild während einer Bildgebung und der Strukturen, die wahrscheinlich eine wesentliche Röntgenstreuung erzeugen, die während der Bildgebung zu vermeiden sind;

Erzeugen einer Röntgenstrahl-Abschwächungskarte **(32)**, die eine Röntgenbildgebungsaussetzung für die bestimmten Strukturen des Subjekts bei der Bildgebung des Patienten während einer Prozedur definiert; und

Bestimmen mindestens einer Röntgenkollimator-Einstellung für den Röntgenkollimator basierend auf der Röntgenstrahl-Abschwächungskarte.

**14.** Verfahren nach Anspruch 13, weiter umfassend:

Erhalten eines Röntgenstreumodells, das mindestens eines von (i) Röntgenstreuung von mindestens einem Gewebe oder anatomischen Strukturen des Subjekts, (ii) Röntgenstreuung von einem Implantat des Subjekts, (iii) räumlichen Informationen in Bezug auf mindestens einen Bereich des Subjekts oder (iv) Informationen zum Verfahrenskontext darstellt; und

Erzeugen der Röntgenstrahl-Abschwächungskarte **(32)** durch Anwenden eines Rechenmodells **(34),** das trainiert ist, um die Röntgenstrahl-Abschwächungskarte basierend auf dem Röntgenstreumodell und mindestens einem Bildmerkmal zu erzeugen.

**15.** Nichtflüchtiges, computerlesbares Speichermedium, das ein gespeichertes Computerprogramm aufweist, das Anweisungen umfasst, die, wenn sie von einem Prozessor ausgeführt werden, den Prozessor veranlassen, das Verfahren nach Anspruch 13 oder 14 zu betreiben.

**Revendications**

**1.** Système **(1)** pour fournir des réglages de collimateur à rayons X, le système **(1)** comprenant :
un contrôleur **(14)** comprenant un processeur en communication avec une mémoire, le processeur étant configuré pour :

recevoir une image de rayons X d'un sujet à partir d'un dispositif d'imagerie par rayons X, dans lequel le dispositif d'imagerie par rayons X **(12)** inclut un collimateur à rayons X (10) configuré pour collimater un faisceau de rayons X du dispositif d'imagerie par rayons Xpour imager le sujet ;

identifier, dans l'image de rayons X, au moins une caractéristique d'image indicative d'au moins une caractéristique d'exposition aux rayons X du sujet ;

déterminer, sur l'image de rayons X, les structures d'intérêt pour l'exposition aux rayons X pendant une imagerie et les structures susceptibles de produire une diffusion importante des rayons X à éviter pendant une imagerie ;

générer une carte d'atténuation de faisceau de rayons X **(32)** définissant une exposition d'imagerie par rayons X pour les structures déterminées du sujet lors de l'imagerie du patient au cours d'une procédure ; et

déterminer au moins un réglage de collimateur à rayons X sur la base de la carte d'atténuation de faisceau de rayons X.

**2.** Système (1) selon la revendication 1, dans lequel la carte d'atténuation de faisceau de rayons X **(32)** définit une ou plusieurs valeurs d'atténuation de faisceau de rayons X cible correspondant à un ou plusieurs pixels représentant les une ou plusieurs régions du sujet.

**3.** Système **(1)** selon la revendication 1, dans lequel au moins une caractéristique d'exposition aux rayons X du sujet comprend au moins l'une des suivantes : (i) l'emplacement d'un type de tissu du sujet, (ii) l'emplacement d'une structure anatomique à l'intérieur du sujet, (iii) des informations spatiales sur l'anatomie du sujet ou (iv) l'emplacement d'un implant à l'intérieur du sujet.

**4.** Système **(1)** selon la revendication 1, dans lequel le processeur est en outre configuré pour :

obtenir un modèle de diffusion de rayons X représentant au moins un des éléments suivants : (i) la diffusion de rayons X provenant d'au moins un parmi un tissu ou des structures anatomiques du sujet, (ii) la diffusion de rayons X provenant d'un implant du sujet, (iii) des informations spatiales relatives à l'au moins une région du sujet ou (iv) des informations contextuelles procédurales ; et

générer la carte d'atténuation de faisceau de rayons X **(32)** par application d'un modèle de calcul **(34)** entraîné à générer la carte d'atténuation du faisceau de rayons X sur la base du modèle de diffusion de rayons X et d'au moins une caractéristique d'image.

5.  Système **(1)** selon la revendication 4 :

    dans lequel le modèle de calcul est en outre entraîné à générer une carte de confiance **(36)** définissant une ou plusieurs valeurs de confiance pour les une ou plusieurs valeurs d'atténuation de faisceau de rayons X de la carte d'atténuation du faisceau de rayons X ; et

    dans lequel le processeur est en outre configuré pour déterminer l'au moins un réglage de collimateur à rayons X sur la base de la carte de confiance d'atténuation de faisceau de rayons X.

6.  Système **(1)** selon la revendication 5, dans lequel :
    la carte d'atténuation de faisceau de rayons X **(32)** définit les une ou plusieurs valeurs d'atténuation de faisceau de rayons X cible correspondant à un ou plusieurs pixels représentant les une ou plusieurs régions du sujet ; et

    la carte de confiance **(32)** définit une ou plusieurs valeurs de confiance correspondant aux un ou plusieurs pixels représentant les une ou plusieurs régions du sujet.

7.  Système **(1)** selon la revendication 1, dans lequel le processeur en outre configuré pour :
    effectuer une segmentation automatique de l'image de rayons X afin d'identifier au moins une caractéristique de l'image.

8.  Système (1) selon la revendication 1, dans lequel le processeur est en outre configuré pour :
    ajuster le collimateur à rayons X **(10)** à l'au moins un réglage de collimateur à rayons X.

9.  Système (1) selon la revendication 1, dans lequel le processeur est configuré pour :

    générer la carte d'atténuation de faisceau de rayons X **(32)** pour inclure une ou plusieurs valeurs de pixels non binaires ; et

    déterminer l'au moins un réglage de collimateur à rayons X pour fournir une atténuation partielle de rayons X en correspondance avec les valeurs de pixels non binaires de la carte d'atténuation de faisceau de rayons X **(32).**

10. Système **(1)** selon la revendication 4, comprenant en outre un second processeur configuré pour entraîner le modèle de calcul, le second processeur étant configuré pour :

    recevoir des images de rayons X acquises précédemment à partir d'au moins une procédure antérieure ; annoter des régions d'intérêt (ROI) dans les images de rayons X acquises précédemment;

    attribuer un score indiquant l'importance de montrer ou de collimater chaque ROI annotée ; et

    générer le modèle informatique basé sur la ROI annotée et le score attribué.

11. Système **(1)** selon la revendication 1, comprenant en outre le dispositif d'imagerie à rayons X **(12)** incluant le collimateur à rayons X (**10**).

12. Système **(1)** selon la revendication 11, dans lequel le collimateur à rayons X **(10)** comprend un collimateur multilames (MLC) et au moins un réglage de collimateur à rayons X est au moins un réglage de lame de collimateur du MLC.

13. Procédé de fourniture de réglages de collimateur à rayons X, le procédé comprenant :

    la réception d'une image de rayons X d'un sujet à partir d'un dispositif d'imagerie par rayons X, dans lequel le dispositif d'imagerie par rayons X **(12)** inclut un collimateur à rayons X **(10)** configuré pour collimater un faisceau de rayons X du dispositif d'imagerie par rayons X pour imager le sujet ;

    l'identification, dans l'image de rayons X, d'au moins une caractéristique d'image indicative d'au moins une caractéristique d'exposition aux rayons X du sujet ;

    la détermination, sur l'image de rayons X, des structures d'intérêt pour une exposition aux rayons X pendant l'imagerie et des structures susceptibles de produire une diffusion importante des rayons X à éviter pendant l'imagerie ;

la génération d'une carte d'atténuation de faisceau de rayons X **(32)** définissant une exposition d'imagerie par rayons X pour les structures déterminées du sujet lors de l'imagerie du patient au cours d'une procédure ; et

la détermination d'au moins un réglage de collimateur à rayons X pour le collimateur à rayons X sur la base de la carte d'atténuation de faisceau de rayons X.

14. Procédé selon la revendication 13, comprenant en outre :

l'obtention d'un modèle de diffusion de rayons X représentant au moins un élément parmi (i) la diffusion de rayons X provenant d'au moins un tissu ou une structure anatomique du sujet, (ii) la diffusion de rayons X provenant d'un implant du sujet, (iii) des informations spatiales relatives à l'au moins une région du sujet ou (iv) des informations contextuelles procédurales ; et

la génération de la carte d'atténuation de faisceau de rayons X (32) par application d'un modèle de calcul **(34)** entraîné à générer la carte d'atténuation de faisceau de rayons X sur la base du modèle de diffusion de rayons X et de l'au moins une caractéristique d'image.

15. Support de stockage non transitoire lisible par ordinateur présentant un programme informatique comprenant des instructions qui, lorsqu'elles sont exécutées par un processeur, amènent le processeur à exécuter le procédé selon la revendication 13 ou 14.

Monitoring
method or process
102

34

32    36

18

24

28

14

22

20

30

26

1

8

6

9

16

17

12

10

19

FIG. 1

FIG. 2

FIG. 3

FIG. 4

**REFERENCES CITED IN THE DESCRIPTION**

**Patent documents cited in the description**

- DE 102011087590
- US 2015272531 A
- US 9280837 B
- US 2021045707 A